Europäisches Patentamt

**European Patent Office**

**Office européen des brevets**

(19)

(11) Publication number: **0 013 785**
A1

(12)

# EUROPEAN PATENT APPLICATION

(21) Application number: **79300101.7**

(22) Date of filing: **19.01.79**

(51) Int. Cl.³: **C 07 D 307/77**
// C07C79/46, C07C76/02

(43) Date of publication of application: **06.08.80**
**Bulletin 80/16**

(84) Designated Contracting States: **BE DE FR GB NL**

(71) Applicant: **GULF RESEARCH & DEVELOPMENT COMPANY, P.O. Box 2038, Pittsburgh Pennsylvania 15230 (US)**

(72) Inventor: **Schulz, Johann Gustav D., 201 Conover Road, Pittsburgh Pennsylvania 15208 (US)**

(74) Representative: **Fitzpatrick, Alan James et al, FITZPATRICKS 14-18 Cadogan Street, Glasgow, G2 6QW (GB)**

(54) Mixture of polycyclic aromatic polycarboxylic anhydrides carrying nuclear nitro groups, and their preparation.

(57) A novel mixture of polycyclic aromatic polycarboxylic anhydrides carrying nuclear nitro groups which is substantially soluble in acetone but substantially insoluble in water is prepared by a process which comprises nitrating a slurry of carbonaceous material with nitric acid, recovering solids from the nitration product extracting an acetone-soluble mixture of carboxylic acids from the solids and dehydrating the mixture to obtain the anhydrides.

EP 0 013 785 A1

TITLE MODIFIED,

see front page  AROMATIC ANHYDRIDES

BACKGROUND OF THE INVENTION

## 1. Field of the Invention

This invention relates to a mixture of polycyclic aromatic polycarboxylic anhydrides carrying nuclear nitro groups that is substantially soluble in acetone but substantially insoluble in water and a process for preparing the mixture by treating a carbonaceous material with nitric acid and dehydrating the resulting product.

## 2. Description of the Prior Art

Aromatic anhydrides, such as benzophenone-3,4,3',4'-tetracarboxylic dianhydride and pyromellitic anhydrides, are useful compounds as curing agents, for example, in combination with epoxy resins. These materials, however, are costly and it would be desirable to prepare aromatic anhydrides that would similarly be useful as curing agents but that would be inexpensive.

## SUMMARY OF THE INVENTION

I have found a curing agent that is both effective and inexpensive to produce, which curing agent is composed of a mixture of polycyclic aromatic polycarboxylic anhydrides carrying nuclear nitro groups that is substantially soluble in acetone but substantially insoluble in water obtained as a result of the nitric acid oxidation of a carbonaceous material, such as coal, followed by dehydration. The precursor polycyclic aromatic polycarboxylic acid mixture can be prepared in accordance with the procedure defined and claimed in U.S. Application Serial No. 696,752, filed concurrently herewith by J. G. D. Schulz and E. T. Sabourin entitled Organic Acids and Process for Preparing Same, now U.S. Patent No. 4,052,448, dated October 4, 1977.

-2-

The individual components of the mixture of polycyclic aromatic polycarboxylic anhydrides claimed herein are
believed to be composed of condensed and/or non-condensed
benzene rings, with an average number of benzene rings in the
individual molecules ranging from two to about ten, but
generally from three to about eight. On the average, the
number of carboxyl groups carried by the individual molecules
will range from about zero to about eight, generally from
about zero to about six, the average number of anhydride
groups carried by the individual molecules will range from
about one to about five, generally from about one to about
four, and the average number of nitro groups from about one
to about four, generally from about two to about three. The
average molecular weight of the mixture will range from about
600 to about 1500, generally from about 700 to about 1000, and
the average neutral equivalent will range from about 80 to
about 200, generally from about 100 to about 150.

A preferred procedure for obtaining the above mixtures is described as follows. There is introduced into a
reactor an aqueous solution of nitric acid and a carbonaceous
material. The nitric acid can have a concentration of about
five to about 90 per cent, but preferably will be in the range
of about 10 to about 70 per cent. The carbonaceous material
is preferably a solid in the form of a slurry, for example, an
aqueous slurry containing the carbonaceous material in particulate form and from about 50 to about 90 weight per cent
of water.

The solid carbonaceous material that can be used herein can have the following composition on a moisture-free
basis:

TABLE I

| | Weight Per Cent | |
| | Broad Range | Preferred Range |
|----------|-------------|-----------------|
| Carbon | 45 – 95 | 60 – 92 |
| Hydrogen | 2.5 – 7 | 4 – 6 |
| Oxygen | 2.0 – 45 | 3 – 25 |
| Nitrogen | 0.75 – 2.5 | 0.75 – 2.5 |
| Sulfur | 0.3 – 10 | 0.5 – 6 |

-3-

The carbon and hydrogen content of the carbonaceous material will reside primarily in multi-ring aromatic compounds (condensed and/or uncondensed), heterocyclic compounds, etc. Oxygen and nitrogen are believed to be present primarily in chemical combination. Some of the sulfur is believed to be present in chemical combination with the aromatic compounds and some in chemical combination with inorganic elements associated therewith, for example, iron and calcium.

In addition to the above the solid carbonaceous material being treated herein will also contain solid, primarily inorganic, compounds which will not be converted to the desired organic mixture, which are termed ash, and are composed chiefly of compounds of silicon, aluminum, iron and calcium, with smaller amounts of compounds of magnesium, titanium, sodium and potassium. The ash content of the carbonaceous material treated will amount to less than about 50 weight per cent, based on the moisture-free carbonaceous material, but, in general, will amount to about 0.1 to about 30 weight per cent, usually about 0.5 to about 20 weight per cent.

Anthracitic, bituminous and subbituminous coal, lignitic materials, and other type of coal products referred to in ASTM D-388 are exemplary of the solid carbonaceous materials which can be treated to produce the organic mixture. Some of these carbonaceous materials in their raw state will contain relatively large amounts of water. These can be dried prior to use herein. The carbonaceous material, prior to use, is preferably ground in a suitable attrition machine, such as a hammermill, to a size such that at least about 50 per cent of the carbonaceous material will pass through a 40-mesh (U.S. Series) sieve. As noted, the carbonaceous material is slurried in a suitable carrier, preferably water, prior to reaction with nitric acid. If desired, the carbonaceous material can be treated, prior to reaction herein, using any conventional means, to remove therefrom any materials forming a part thereof that will not be converted in reaction with nitric acid herein.

The reactant mixture in the reactor is stirred while being maintained at a temperature of about 15° to about 200°C., preferably about 50° to about 100°C., and a pressure of about

-4-

atmospheric to about 1000 pounds per square inch gauge (about atmospheric to about 70 kilograms per square centimeter), preferably about atmospheric to about 500 pounds per square inch gauge (about atmospheric to about 35 kilograms per square centimeter) for about 0.5 to about 15 hours, preferably about two to about six hours. In order to obtain the desired mixture without losing appreciable amounts of carboxyl and/or nitro groups on the acids that are formed during the oxidation, and to obtain the desired acids in high yields in the reactor, it is absolutely critical that the reaction conditions therein, namely nitric acid concentration, temperature, pressure and reaction time, be so correlated to minimize and, preferably, to avoid decarboxylation and denitrofication. Gaseous products, such as nitrogen oxides, can be removed from reactor by any suitable means.

The reaction product removed from the reactor is found to be soluble, or reactable with, sodium hydroxide. At this point it is necessary to separate the oxidized product from the water and nitric acid associated therewith. This separation must be accomplished in a manner so that the carboxyl and nitro groups are not removed from the acid product. Distillation for the removal of water will not suffice, because under the conditions required for such separation, a significant loss of carboxyl groups and nitro groups would occur. A mechanical separation will, however, suffice. The reaction product is therefore led to a first separator, which can be, for example, a filter or a centrifuge.

The solids that are recovered in the separator, also soluble in sodium hydroxide, are led to a second separator wherein they are subjected to extraction with acetone. Such separation can be carried out at a temperature of about 20° to about 60°C., preferably about 25° to about 50°C., and a pressure of about atmospheric to about 500 pounds per square inch gauge (about atmospheric to about 35 kilograms per square centimeter), preferably about atmospheric to about 100 pounds per square inch gauge (about atmospheric to about seven kilograms per square centimeter). The solid material, insoluble in acetone, is removed from the latter separator by a first line and the acetone solution of the acid mixture by a second line. The acetone solution is then led to drier wherein acetone is separated therefrom and an acetone-soluble, water-insoluble polyaromatic, polycarboxylic acid mixture is recovered. As before, the acid mixture in the drier must be treated by so correlating the conditions therein to remove acetone therefrom in such manner so as to minimize and, preferably, avoid, decarboxylation and denitrofication. The temperature can be in the range of about 10° to about 60°C., preferably about 20° to about 50°C., the pressure about 10 millimeters of mercury to about atmospheric, preferably about 30 millimeters of mercury to about atmospheric, for about 0.5 to about 25 hours, preferably about one to about five hours.

Although it has been stated above that the acid mixture is acetone-soluble and acetone has been shown as suitable in the process, this has been done merely as a characterization of the composition and to exemplify one embodiment of the process. Many polar solvents can be used in place of acetone. Among the polar solvents that have been used are methanol, ethanol, isopropanol, methyl ethyl ketone, tetrahydrofuran, dioxane, etc. The use of such solvents, therefore, falls within the scope of the invention claimed herein.

The filtrate obtained in the first separator will contain water, nitric acid and most of the inorganic material (ash) that was present in the carbonaceous charge. In addition there can also be present other oxidized materials, which are primarily acetone-soluble, water-soluble organic acids.

In the final stage the acetone-soluble, water-insoluble polyaromatic, polycarboxylic acid mixture is then subjected to dehydration, using any known or convenient procedure, to obtain the corresponding anhydride mixture claimed herein. If desired, the dehydration can be effected using well-known chemical dehydration agents, such as acetic anhydride, anhydrous magneisum, sodium sulfate, etc. In such case the mixture of acids is mixed with from about one to about 80 weight per cent, preferably about ten to about 50 weight per cent, of the dehydrating agent, at a temperature on the order of about 50° to about 250°C., preferably about 100° to about 200°C., for a time sufficient to remove chemically water from the acid mixture, for example a period of about 0.5 to about 10 hours, preferably about one to about five hours. Dehydration of the acid mixture to obtain the desired corresponding anhydride mixture can also be effected by heating the mixture without a dehydrating agent at a temperature of about 50° to about 250°C., preferably about 100° to about 200°C. and atmospheric pressure for about 0.5 to about 10 hours, preferably about one to about five hours. This heating is preferably carried out using a carrier, such as p-xylene, dixylethane, toluene, mineral oil, or other inert solvents which will preferably form a low-boiling azeotrope with water, easily removed from the reaction zone during heating.

DESCRIPTION OF PREFERRED EMBODIMENTS

Several runs were carried out in which a North Dakota lignite analyzing as follows, on a substantially moisture-free basis, was subjected to oxidation using nitric acid as the oxidant: 65.03 weight per cent carbon, 4.0 weight per cent hydrogen, 27.0 weight per cent oxygen, 0.92 weight per cent sulfur, 0.42 weight per cent nitrogen and 0.04 weight per cent moisture. The ash was further analyzed and found to contain 43 weight per cent oxygen, 7.8 weight per cent sulfur and the remainder metals.

In each of Runs Nos. 1 to 3, the data of which are summarized below in Table III, 70 per cent aqueous nitric acid was used. In Runs Nos. 2 and 3 over a period of two hours 100 milliliters of the defined nitric acid was gradually added to the stirred slurry containing 100 grams of powdered lignite defined above (corresponding to 67.5 grams of moisture-free feed) and 370 grams of water while maintaining the contents at selected temperature levels and atmospheric pressure. In Run No. 1, otherwise identical to Runs Nos. 2 and 3, a five-hour reaction time was employed. Nitrogen oxides were permitted to escape from the reaction zone as they evolved.

At the end of the reaction period the product slurry was withdrawn from the reaction zone and filtered to obtain a solids fraction and a filtrate. The solids were extracted with acetone at atmospheric temperature and pressure. The acetone solution was then subjected to evaporation at atmospheric temperature and pressure to obtain the acid mixture.

The acetone insoluble portion was found to be soluble in sodium hydroxide and to comprise organic acids of a relatively lower carboxyl functionality than the acetone-soluble portion.

In each of the runs some acetone soluble, water-soluble organic acids were also found. The work-up of the filtrate was carried out as follows. Initially the filtrate was subjected to distillation to separate unreacted nitric acid and water therefrom. The remaining solids were subjected to extraction with acetone at atmospheric temperature and atmospheric pressure. The acetone solution was dried to remove acetone therefrom, resulting in the recovery of small amounts of the acetone-soluble, water-soluble organic acids completely soluble in sodium hydroxide. The average molecular weight of the mixtures obtained was about 800 and the average neutral equivalent about 110. The residue was mainly ash.

In separate runs, 30 grams of the acetone-soluble mixture recovered above was mixed with 150 grams of mixed xylenes and the resulting mixture was heated to reflux temperature and maintained at such temperature, while stirring, for two hours. In each instance about 2.2 grams of water was evolved, indicating dehydration of the acid mixture. The presence of anhydride in the infra-red spectrum of each product obtained further proved dehydration had taken place. In each instance the neutral equivalent was found to be 119.

In a separate run, 50 grams of another portion of the acetone-soluble mixture obtained in Run No. 3 was mixed with 150 grams of acetic anhydride and 200 grams of tetrahydrofuran as a solvent. The mixture was brought to reflux temperature and maintained at such temperature while stirring, for 16 hours. The product, 24 grams which was found to be totally soluble in the tetrahydrofuran-acetic anhydride mixture, was recovered by evaporation of the solvent at 35°C. The neutral equivalent was found to be 105. The presence of anhydride bands in the infra-red spectrum of the product is indicative of dehydration. The results obtained are tabulated below in Table II.

## TABLE II

| Run No. | Temperature, °C | Reaction Time, Hours | Acetone-Soluble, Water-Insoluble Product, Grams |
|---------|-----------------|----------------------|-------------------------------------------------|
| 1. | 50 | 5 | 67.0 |
| 2. | 70 | 2 | 51.1 |
| 3. | 90 | 2 | 52.5 |

## TABLE II (Cont'd)

Ananysis of Acid Product, Weight Per Cent

| Run No. | Carbon | Hydrogen | Nitrogen | Oxygen | Sulfur | Ash |
|---------|--------|----------|----------|--------|--------|-----|
| 1. | 56.30 | 4.80 | 4.60 | 33.27 | 0.31 | 0.72 |
| 2. | 55.52 | 3.72 | 4.70 | 35.13 | 0.30 | 0.63 |
| 3. | 53.94 | 4.38 | 4.61 | 36.39 | 0.25 | 0.43 |

## TABLE II (Cont'd)

| Run No. | Neutral Equivalent of Anhydride Mixture |
|---------|-----------------------------------------|
| 1. | 119 |
| 2. | 119 |
| 3. | 119,105 |

- 9 -

The anhydride mixtures obtained herein are effective curing agents. This is shown below.

### Example 1

Twenty-five grams of EPON 1004 (Shell Chemical Company), an epoxy resin believed to be the diglycidyl ether of Bisphenol A having an epoxy equivalent of 900, 10.5 grams of the anhydride mixture from Run No. 3, above, prepared using acetic anhydride as a dehydrating agent, and 0.9 gram of tin octanoate as an accellerator intimately mixed, the mixture was added to a ceramic jar and rolled with burundum cylinders for 24 hours. Circular molded discs were prepared at 165°C., 1600 pounds per square inch gauge (112.5 kilograms per square centimeter) and two-hour molding time. The initial Barcol hardness was from 6 to 8. When the discs were post cured overnight at 175°C., the hardness increased to 12 to 16.

### Example II

Nine grams of EPON 1004, 20 grams of the same anhydride mixture used in Example I and 0.9 gram of tin octanoate were ball milled overnight to obtain a powdered mixture. Molded products were prepared by molding the product at 175°C. and a pressure of 1000 to 1200 pounds per square inch gauge (70.5 to 84.4 kilograms per square centimeter) over a period of one-half hour. A good black disc was again prepared. The product was post cured at 175°C. for 16 hours to obtain a final product having a Barcol hardness of 25 to 28.

Obviously, many modifications and variations of the invention, as hereinabove set forth, can be made without departing from the spirit and scope thereof and, therefore, only such limitations should be imposed as are indicated in the appended claims.

CLAIMS

1.    A mixture of polycyclic aromatic polycarboxylic anhydrides carrying nuclear nitro groups that is substantially soluble in acetone but substantially insoluble in water.

2.    The composition of claim 1 wherein the average number of benzene rings in the individual molecules ranges from two to about ten.

3.    The composition of claim 1 wherein the average number of benzene rings in the individual molecules ranges from about three to about eight.

4.    The composition of claim 1 wherein the average number of carboxyl groups carried by the individual molecules ranges from about zero to about eight and the average number of anhydride groups carried by the individual molecules ranges from about one to about five.

5.    The composition of claim 1 wherein the average number of carboxyl groups carried by the individual molecules ranges from about zero to about six and the average number of anhydride groups carried by the individual molecules ranges from about one to about four.

6.    The composition of claim 1 wherein the average number of nitro groups carried by the individual molecules ranges from about one to about four.

7.    The composition of claim 1 wherein the average number of nitro groups carried by the individual molecules ranges from about two to about three.

8.    The composition of claim 1 wherein the average molecular weight of the mixture ranges from about 600 to about 1500.

9.    The composition of claim 1 wherein the average molecular weight of the mixture ranges from about 700 to about 1000.

10.    The composition of claim 1 wherein the average nuclear equivalent ranges from about 80 to about 200.

11.    The composition of claim 1 wherein the average nuclear equivalent ranges from about 100 to about 150.

12.    A process for converting a carbonaceous material to a mixture of polycyclic aromatic polycarboxylic anhydrides carrying nuclear nitro groups that is substantially soluble in acetone but substantially insoluble in water which comprises subjecting a slurry containing carbonaceous material to reaction with aqueous nitric acid, mechanically separating the solids in the resulting slurry, extracting the resulting solids with a polar solvent, separating the acetone from the extract to recover the carboxylic acid mixture, and then subjecting said acid mixture to dehydration to obtain the corresponding anhydride mixture.

13. The process of claim 12 wherein said polar solvent is acetone.

14. The process of claim 12 wherein the nitric acid has a concentration of about 5 to about 90 per cent and the reaction is carried out at a temperature of about 15° to about 200°C. for about 0.5 to about 15 hours.

15. The process of claim 12 wherein the nitric acid has a concentration of about 10 to about 70 per cent and the reaction is carried out at a temperature of about 50° to about 100°C. for about two to about six hours.

16. The process of claim 12 wherein mechanical separation is effected by filtration.

17. The process of claim 12 wherein the acetone separation is effected by subjecting the acetone extract to drying.

18. The process of claim 12 wherein said dehydration is effected by treatment with a chemical dehydrating agent.

19. The process of claim 18 wherein said dehydrating agent is acetic anhydride.

20. The process of claim 12 wherein said dehydration is effected by heating the acid mixture at a temperature of about 50° to about 250°C.

21. The process of claim 12 wherein said dehydration is effected by heating the acid mixture at a temperature of about 100° to about 200°C.

22. The process of claim 12 wherein the carbonaceous material is coal.

European Patent
Office

**EUROPEAN SEARCH REPORT**

Application number

EP 79 30 0101

| DOCUMENTS CONSIDERED TO BE RELEVANT | | | CLASSIFICATION OF THE APPLICATION (Int. Cl.²) |
|---|---|---|---|
| Category | Citation of document with indication, where appropriate, of relevant passages | Relevant to claim | |
| X | US - A - 2 949 350 (HEINZE et al.)<br>* Claim 1; column 2, lines 25-30; column 3, lines 3,4; column 5, example VII; column 6, lines 3, 37-40,54-57 *<br><br>-- | 1,12,<br>13,22 | C 07 D 307/77<br>C 07 C 79/46<br>76/02 |
| X | DE - C - 743 225 (JUETTNER)<br>* Claims 1,2; page 2, lines 10-18; example I, lines 80-95 *<br><br>-- | 12,14,<br>15,22 | |
| D | US - A - 4 052 448 (SCHULZ et al.)<br>* Claims 1-7 *<br><br>---- | 12-22 | TECHNICAL FIELDS SEARCHED (Int.Cl.²)<br><br>C 07 C 79/46<br>C 07 D 307/77<br>C 07 C 76/02 |

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 07-09-1979 | KLAG |

EPO Form 1503.1  06.78